# EUROPEAN PATENT APPLICATION

(11) **EP 3 821 896 A1**
(43) Date of publication of application: **19.05.2021**
(21) Application number: 19834312.1
(22) Date of filing: 09.07.2019
(51) Int. Cl.: A61K 31/7034, A61K 31/122, A61P 17/14, A61K 8/60, A61K 8/35, A61Q 7/00, A61Q 5/00, A61K 8/42, A61K 8/49, A61K 31/16

(54) **COMPOSITION FOR PREVENTING HAIR LOSS OR PROMOTING HAIR REGROWTH**

(30) Priority: 09.07.2018 KR 20180079534; 22.10.2018 KR 20180126283
(71) Applicant: LG Household & Health Care Ltd., Seoul 03184 (KR)
(72) Inventor: SHIN, Jae-Young, Seoul 07795 (KR); LEE, Sang-Hwa, Seoul 07795 (KR); KIM, Jae-Yoon, Seoul 07795 (KR); LEE, So-Young, Seoul 07795 (KR)
(74) Representative: Goddar, Heinz J.
(86) International application number: PCT/KR2019/008438
(87) International publication number: WO 2020/013581

(57) **Abstract**

The present disclosure relates to a composition for preventing alopecia and accelerating hair growth, including an ingredient inducing new development of hair or accelerating hair growth. The composition for preventing alopecia and accelerating hair growth reduces a period of transition from a telogen stage to an anagen stage in the hair growth cycle to accelerate hair growth and to delay a transition to a catagen stage, and thus provides excellent effects of preventing alopecia and accelerating hair growth. In addition, the composition is safe to the human body, causes no side effect and provides excellent effects of accelerating proliferation of dermal papilla cells and enhancing hair elasticity.

## Description

### TECHNICAL FIELD

The present application claims priority to Korean Patent Application No. 10-2018-0079534 filed on July 9, 2018 and Korean Patent Application No. 10-2018-0126283 filed on October 22, 2018 in the Republic of Korea, the disclosures of which are incorporated herein by reference.

The present disclosure relates to a composition for accelerating hair growth. More particularly, the present disclosure relates to a composition for preventing alopecia and accelerating hair growth, including an ingredient inducing new development of hair or hair growth acceleration.

### BACKGROUND ART

As times change, attentions to beauty have been increased gradually and hair occupies an important part thereof. Hair is a thin and keratinized structure produced at the skin surface. Hair has a cushioning effect against external impact, functions to protect the human body from external irritations, such as direct sunlight, cold, friction, risk, or the like and to discharge heavy metals, such as arsenic, mercury, zinc, or the like, harmful to the human body to the outside of the body, and has been spotlighted in terms of beauty and decoration more recently. However, persons suffering from alopecia have been increased due to various causes, such as a change in dietary life and an increase in internal and external stress.

A man has approximately 100,000-150,000 hair strands and each hair has a different growth cycle. The hair growth cycle includes the following three stages: an anagen stage in which hair grows most actively, a catagen stage in which hair starts to be degenerated, and a telogen stage in which hair growth is stopped or takes a break.

In controlling the hair growth cycle, it is known that activities of dermal papilla cells forming hair follicles and outer root sheath cells including hair follicle stem cells and various cytokines and growth factors produced at the cells play an important role. For example, Dickkopf-1 (DKK-1) plays an important role in inhibition and destroy of growth of hair follicles (J Invest. Dermatol,2008:128(2)). Meanwhile, regeneration of hair follicles and activation of stem cells should be performed for the purpose of cyclic hair growth. It is known that such regeneration and activation are performed by production and activation of Wnt/β-catenin (Nature 2007: 447 (7142)).

In general, alopecia refers to a condition of an abnormal increase in depilation caused by a decrease in proportion of hair in an anagen state and an increase in proportion of hair in a catagen stage or a telogen stage in the hair growth cycle. In the case of a normal person, the proportion of hair in an anagen stage is high, while a person suffering from alopecia has a high proportion of hair in a telogen stage and shows a visible alopecia phenomenon. Persons suffering from alopecia are characterized by downsizing of hair. As alopecia proceeds, the period of an anagen stage is decreased and a transition to a catagen stage and a telogen stage is accelerated, and then the volume of dermal papilla is reduced and hair follicles are gradually downsized. Therefore, in order to treat alopecia, it is important to recover hair in a telogen state rapidly to an anagen state and to increase such a decreased anagen stage.

As causes of alopecia, there have been discussed hyperaction of male hormones, excessive secretion of sebum, poor blood circulation, degradation of scalp functions caused by peroxides, bacteria, or the like, hereditary factors, aging, stress, or the like. However, the causes of alopecia have not been shown clearly to date.

Male hormones play an important role in growth and elimination of hair follicles. Among the factors controlled by male hormones in hair follicles, TGF-β plays an important role in inhibiting growth of hair follicles by male hormones and inducing a catagen stage in the hair follicle cycle (Tsuji Y, Denda S, Soma T, Raftery L, Momoi T, Hibino T, A potential suppressor of TGF-beta delays catagen progression in hair follicles, J Investig Dermatol Symp Proc, 2003:8(1):65-68). In other words, apoptosis of hair matrix cells and outer root sheath cells is induced by an increase in TGF-β, since when TGF-β is expressed in hair follicle dermal papilla cells by male hormones, causes apoptosis of hair follicle basal cells and outer root sheath cells and induces a transition of hair follicles in an anagen stage to a catagen stage. Therefore, hair follicles in a telogen stage are increased, and hair follicles in an anagen stage are changed into hair follicles in a catagen stage, and thus hair is eliminated easily and thin and short hair is developed (Young Ju, Lee, Ju Young, Lee, Clinical Diagnosis and Management of Patients Suffering from Male Androgenic Alopecia, Journal of the Korean Beauty Society, 2007: 13(2): 799-810). Therefore, it is known that TGF-β inhibits growth of hair follicles.

Escin is a saponin-based material contained in Marronnier extract in a large amount, and is used largely as an anti-inflammatory agent, vascular contracting agent or vascular protecting agent. However, before the present disclosure, it is not known that escin has effects of improving anti-alopecia and accelerating hair growth.

Agents developed to date for treating or preventing alopecia include those based on female hormones for accelerating blood circulation, reinforcing hair root functions, moisturizing the scalp and inhibiting male hormones, agents containing minoxidil, finasteride, trichosaccharide, or the like. A typical agent for treating alopecia through application, minoxidil (2,4-diamino-6-piperidinopyrimidine-3-oxide), increases the amount of blood flow toward the scalp. In addition, an oral administration agent for treating alopecia, finasteride, inhibits the activity of 5α-reductase to reduce production of DHT, an active male hormone. However, the above-mentioned agents have many limitations in their use due to their side effects or precautions for use.

### DISCLOSURE

### Technical Problem

The present disclosure is directed to providing a composition for preventing alopecia or accelerating hair growth.

The present disclosure is also directed to providing a hair or scalp care product including the composition for preventing alopecia or accelerating hair growth.

### Technical Solution

The inventors of the present disclosure have conducted many studies to improve the problems, such as side effects and precautions for use, of conventional alopecia-treating agents and to develop a composition which works effectively for accelerating hair growth as well as is safe to the human body without any disadvantage, such as a low effect of preventing alopecia and/or accelerating hair growth. As a result, the inventors have found that escin has an excellent effect of preventing alopecia and accelerating hair growth. The present disclosure is based on this finding.

Particularly, the inventors of the present disclosure have found that escin accelerates the activity of the Wnt/β-catenin signal transmission system in dermal papilla cells, accelerates proliferation of dermal papilla cells and increases expression of β-catenin. In other words, it has been found through an in-vitro test that escin plays an important role in changing the physiological cycle of hair from a catagen stage to an anagen stage by amplifying Wnt/β-catenin signal transmission. In addition, the inventors have found that when a patient suffering from alopecia is treated with a composition for treating alopecia including escin, there is provided a significantly excellent effect of improving thickness, degree of crowding and elasticity of hair. Further, when carrying out a comparative test with a commercially available product, minoxidil, the composition for treating alopecia including escin according to the present disclosure shows a similar or higher effect of preventing alopecia and accelerating hair growth, as compared to minoxidil.

In one aspect of the present disclosure, there is provided a composition for preventing alopecia or accelerating hair growth, including escin as an active ingredient.

According to an embodiment, the content of escin used as an active ingredient of the composition for preventing alopecia or accelerating hair growth according to the present disclosure may be 0.001-10 wt%, particularly 0.01-5 wt%, and more particularly 0.1-2 wt%, based on the total weight of the composition. According to the present disclosure, when the content of escin is 0.001-10 wt% based on the total weight of the composition, it is shown that there is provided an excellent effect of preventing alopecia and accelerating hair growth in vitro or clinically. When the content of escin is less than 0.001 wt%, it is not possible to provide a sufficient effect of accelerating hair growth. When the content of escin is larger than 10 wt%, formulation stability may be degraded and skin irritation may occur. More particularly, the content of escin may be 0.5-2 wt% based on the total weight of the composition. Most particularly, the content of escin may be 1-2 wt% based on the total weight of the composition. According to the present disclosure, it is shown experimentally that a significantly excellent effect of accelerating hair growth and preventing alopecia can be provided within the above-defined range of escin content.

According to the present disclosure, escin may be used as an agent for preventing alopecia or accelerating hair growth in the composition or formulation.

In another aspect of the present disclosure, there is also provided a composition for preventing alopecia or accelerating hair growth, including at least one compound selected from rhaponticin, rhein, chrysophanol and physicon-8-O-d-glucopyranoside, or a salt, hydrate or solvate thereof, as an active ingredient.

Rhaponticin is a stilbenoid glucoside compound, is known to have a potential in treating diabetes mellitus and Alzheimer' disease, and is represented by the following chemical formula:

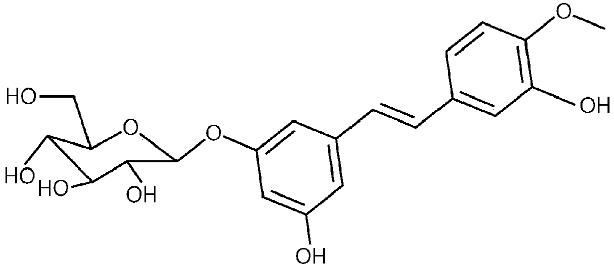

Rhein is a compound that belongs to the class of anthraquinones, is also referred to as cassic acid, is known to have antibacterial and antibiotic properties, and is represented by the following chemical formula:

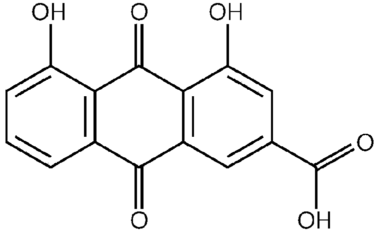

Chrysophanol is a compound that belongs to the class of naturally occurring anthraquinones isolated from fungi and is represented by the following chemical formula:

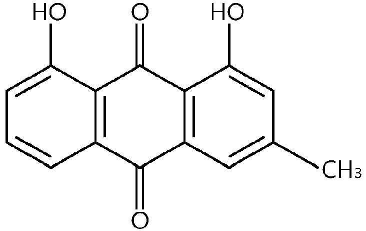

Physicon-8-O-d-glucopyranoside is a naturally occurring anthraquinone derivative and is represented by the following chemical formula:

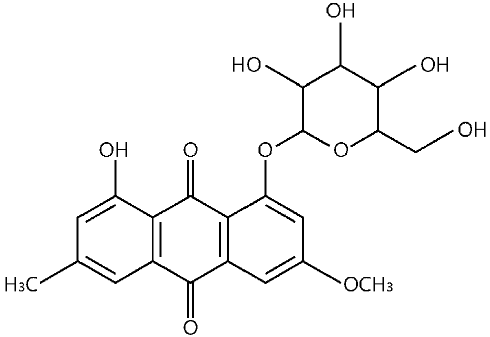

According to an embodiment, the active ingredient according to the present disclosure may include at least one compound, particularly at least two compounds, selected from the above-mentioned compounds. When two or more compounds are used in combination, there is provided a synergic effect with reference to prevention of alopecia and acceleration of hair growth.

According to an embodiment, the active ingredient according to the present disclosure may include at least two compounds which may be a combination of (i) chrysophanol with (ii) any one compound selected from rhaponticin, rhein and physicon-8-O-d-glucopyranoside.

According to another embodiment, the active ingredient according to the present disclosure may include at least two compounds which may be a combination of (i) rhaponticin with (ii) any one compound selected from anthraquinone derivative compounds (rhein, chrysophanol and physicon-8-O-d-glucopyranoside).

According to still another embodiment, the active ingredient according to the present disclosure may include at least two compounds which may be a combination of the four compounds of rhaponticin, rhein, chrysophanol and physicon-8-O-d-glucopyranoside.

In addition, not only the above-mentioned compounds themselves but also salts, hydrates or solvates thereof may be used as active ingredients of the composition according to the present disclosure.

The salts may include those formed by using inorganic acids, such as hydrochloride, hydrobromide and hydroiodide, and those formed by using organic acids, such as acetate, adipate, alginate, aspartate, benzoate, benzenesulfonate, p-toluene sulfonate, bisulfate, sulfamate, sulfate, naphthylate, butyrate, citrate, camphorate, camphorsulfonate, cyclopentanepropionate, digluconate, dodecylsulfate, ethanesulfonate, fumarate, glucoheptanoate, glycerophosphate, hemisulfate, heptanoate, hexanoate, 2-hydroxyethanesulfate, lactate, maleate, methanesulfonate, 2-naphthalenesulfonate, nicotinate, oxalate, tosylate and undecanoate.

Hydrates refer to materials formed by addition of water to the above-mentioned compounds or introduction of water as an ingredient of molecules, and solvates refer to high-order compounds formed between solute molecules or ions and solvent molecules or ions. The hydrates or solvates may be formed by using the above-mentioned acids.

The compound according to the present disclosure includes an effective amount of at least one of the above-mentioned compounds, or a salt, solvate or hydrate thereof, and thus provides an effect of accelerating proliferation of dermal papilla cells and/or enhancing elasticity of hair.

The term 'effective amount' means an amount sufficient to prevent, improve and treat alopecia. The effective amount may be varied suitably with severity of disease, age, body weight, health condition and sex of a patient, administration route, treatment period, or the like.

Particularly, in the composition according to the present disclosure, the effective amount may be 0.00001-50 wt%. Particularly, the composition according to the present disclosure may include rhaponticin in an amount of 0.00001-50 wt%, preferably 0.0001-0.1 wt%, rhein in an amount of 0.00001-50 wt%, preferably 0.0001-01 wt%, chrysophanol in an amount of 0.000001-50 wt%, preferably 0.0001-0.01 wt%, or physicon-8-O-glucopyranoside in an amount of 0.00001-50 wt%, preferably 0.0001-0.1 wt%.

When rhaponticin, rhein, chrysophanol or physicon-8-O-d-glucopyranoside is used in an amount of less than 0.00001 wt%, it is not possible to provide a sufficient effect of accelerating hair growth. When rhaponticin, rhein, chrysophanol or physicon-8-O-d-glucopyranoside is used in an amount of larger than 50 wt%, formulation stability is degraded undesirably.

According to an embodiment, the composition for preventing alopecia or accelerating hair growth may be used as a pharmaceutical composition, non-medical composition or a cosmetic composition. The composition according to the present disclosure may be formulated generally into any form applicable to the skin and particularly formulated into an agent for external use on skin. For example, the composition according to the present disclosure may be prepared into a formulation applicable to the skin, such as liquid, cream, paste or solid.

According to an embodiment of the present disclosure, the composition may be a pharmaceutical composition for preventing or treating alopecia. In this case, the composition for preventing alopecia or accelerating hair growth may include at least one of the above-mentioned compounds, a salt, hydrate or solvate thereof, in combination with at least one pharmaceutically acceptable carrier, vehicle or diluent.

Herein, 'pharmaceutically acceptable' refers to a composition which is physiologically accepted and generally causes no allergic response, such as gastrointestinal disorder or dizziness, or a similar response, when it is administered to the human.

Particular examples of the carrier, vehicle and diluent include lactose, dextrose, sucrose, sorbitol, mannitol, xylitol, erythritol, maltitol, starch, acacia gum, alginate, gelatin, calcium phosphate, calcium silicate, cellulose, methyl cellulose, polyvinyl pyrrolidone, water, methyl hydroxybenzoate, propyl hydroxybenzoate, talc, magnesium stearate and mineral oil. In addition, the composition may further include a filler, anti-agglomerating agent, lubricant, wetting agent, fragrance, emulsifier, preservative, or the like.

According to another embodiment of the present disclosure, the composition may be a non-medical composition. In this case, the active ingredient according to the present disclosure may be added as it is or in combination with other additional non-medical ingredients, and may be used suitably in the conventional manner. Such additional ingredients may be selected and combined optionally depending on formulation or purpose of use of a non-medical product. For example, such additional ingredients may include conventional adjuvants, such as a thickener, stabilizer, solubilizing agent, vitamin, pigment and fragrance, and carriers.

According to another embodiment of the present disclosure, the composition may be a cosmetic composition for preventing or improving alopecia or accelerating hair growth.

When the formulation according to the present disclosure is liquid, a solvent, solubilizing agent or emulsifier may be used as a carrier ingredient. For example, water, ethanol, isopropanol, ethyl carbonate, ethyl acetate, benzyl alcohol, benzyl benzoate, propylene glycol, 1,3-butylglycol oil, glycerol aliphatic ester, polyethylene glycol or sorbitan fatty acid ester, etc. may be used.

When the formulation according to the present disclosure is paste, cream or gel, animal oil, vegetable oil, wax, paraffin, starch, tragacanth, cellulose derivative, polyethylene glycol, silicone, bentonite, talc, zinc oxide, etc. may be used as a carrier ingredient.

When the formulation according to the present disclosure is powder or spray, lactose, talc, silica, aluminum hydroxide, calcium silicate, polyamide powder, etc. may be used as a carrier ingredient. Particularly, in the case of a spray formulation, it may further include a propellent, such as chlorofluorohydrocabon, propane/butane or dimethyl ether.

The ingredients contained in the composition according to the present disclosure may further include those used conventionally for an agent for external use applicable to the skin, besides the active ingredient. For example, the composition may further include at least one additive selected from the group consisting of water, surfactant, moisturizing agent, lower alcohol, chelating agent, sterilizing agent, anti-oxidant, preservative, colorant, fragrance, thickener, polishing agent, sweetening agent, pH modifier, binding agent, foaming agent, solubilizing agent, vitamin, pigment, or the like.

In still another aspect of the present disclosure, there is provided a hair or scalp care product including the composition for preventing alopecia or accelerating hair growth. The hair or scalp care product may be any one selected from the group consisting of a hair growth treatment, scalp clinic agent, scalp scaling agent, scalp massaging agent, scalp care agent, cleaner, shampoo, tonic, hair conditioner, hair lotion, gel, pack, cream, essence, powder, spray, oil, soap, ointment, hair styling agent, hair dye and hair perm agent, but is not limited thereto.

According to an embodiment of the present disclosure, the composition for preventing alopecia or accelerating hair growth is prepared into a formulation of hair tonic or hair lotion (Examples 1-13).

The composition for accelerating hair growth according to the present disclosure may be used preferably through a transdermal administration route, such as direct application or spraying to the skin.

Herein, the term 'administration' refers to introduction of the composition according to the present disclosure through any suitable method. The administration route of the composition according to the present disclosure may be any general route, as long as it allows the composition to access to a desired tissue. Preferably, the composition may be administered through a transdermal route, local application being most preferred. The number of application of the composition according to the present disclosure may be determined by prescription, demand or requirement.

The administration dose of the composition according to the present disclosure may be controlled suitably depending on personal characteristics, such as age, severity of lesion, etc., or formulations. In general, the composition may be administered preferably by applying an adequate amount of the composition to the scalp once or several times per day for 1 week to several months. In the following examples, Composition 1 for treating alopecia is used five times per week for 6 months. As a result, it is determined that the composition shows an excellent effect of accelerating hair growth (Experimental Example s 4 and 8).

### Advantageous Effects

The composition for preventing alopecia and accelerating hair growth according to the present disclosure reduces a period of transition from a telogen stage to an anagen stage in the hair growth cycle to accelerate hair growth and to delay a transition to a catagen stage, and thus provides excellent effects of preventing alopecia and accelerating hair growth.

In addition, the composition according to the present disclosure is safe to the human body, causes no side effect, and provides excellent effects of preventing alopecia and accelerating hair growth. Further, the composition for preventing alopecia or accelerating hair growth according to the present disclosure works effectively in accelerating proliferation of dermal papilla cells and enhancing hair elasticity.

### DESCRIPTION OF DRAWINGS

FIG. 1 illustrates the results of analysis about the effect of amplifying activity of Wnt/β-catenin promoter of escin in dermal papilla cells.
FIG. 2 illustrates the results of the test for determining an increase in expression of β-catenin caused by escin in dermal papilla cells.

### BEST MODE

Hereinafter, the present disclosure will be explained in more detail with reference to exemplary embodiments. This disclosure may, however, be embodied in many different forms and should not be construed as limited to the exemplary embodiments set forth therein. It will be apparent that these exemplary embodiments are provided so that the present disclosure will be complete and understood easily by those skilled in the art.

### Preparation Example 1. Composition 1 for Treating Alopecia/Accelerating Hair Growth (Hair Tonic)

Hair tonics including escin according to Examples 1-6 and Comparative Example 1 were prepared in the conventional manner by using the formulations as shown in the following Table 1.

**[Table 1]**

| Ingredients | Weight ratio (%) | | | | | | |
|---|---|---|---|---|---|---|---|
| | Comp. Ex. 1 | Ex. 1 | Ex. 2 | Ex. 3 | Ex. 4 | Ex. 5 | Ex. 6 |
| Ethanol | 55 | 55 | 55 | 55 | 55 | 55 | 55 |
| Castor Oil | 5 | 5 | 5 | 5 | 5 | 5 | 5 |
| Glycerin | 3 | 3 | 3 | 3 | 3 | 3 | 3 |
| Escin | - | 0.01% | 0.1% | 0.5% | 1% | 2% | 5% |
| Fragrance and Colorant | q.s. | q.s. | q.s. | q.s. | q.s. | q.s. | q.s. |
| Purified Water | Balance (Total 100) | | | | | | |

### Preparation Example 2. Composition 2 for Treating Alopecia/Accelerating Hair Growth (Hair Lotion)

Hair lotion including escin according to Example 7 was prepared in the conventional manner by using the formulation as shown in the following Table 2.

**[Table 2]**

| Ingredients | Weight ratio (%) |
|---|---|
| Cetostearyl Alcohol | 2 |
| Stearyltriethylammonium Chloride | 2 |
| Hydroxyethyl Cellulose | 0.5 |
| Escin | 2 |
| Fragrance and Colorant | q.s. |
| Purified Water | Balance (Total 100) |

### Experimental Example 1. Effect of Controlling Signals (Wnt/β-catenin) Activated in Dermal Papilla Cells

In general, Wnt/β-catenin signals activated in dermal papilla cells are generated while a transition from a catagen stage to an anagen stage occurs in the hair growth cycle, i.e. during a period from beginning of hair growth to an activation stage of hair growth. In addition, in a telogen stage and catagen stage, Wnt/β-catenin signals become weak or disappear to cause degradation of hair follicles and depilation. In this Experimental Example , it is determined how escin contributes to amplification of Wnt/β-catenin signals.

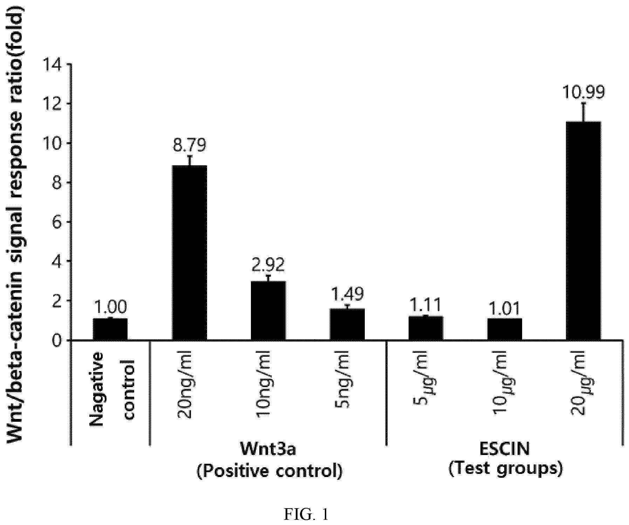

As a positive control which amplifies Wnt/β-catenin signals, wnt3a protein was used. In addition, dimethyl sulfoxide (DMSO) was used as a negative control. In a 96-well culture plate, approximately 3x10⁴ of Wnt reporter HEK293SA cells were seeded to each well and treatment with escin was carried out at a concentration of 5 µg/mL, 10 µg/mL and 20 µg/mL. Then, a luciferase assay kit (E1960) available from Promega Corp. was used to carry out reporter analysis according to the test method provided by the company. In addition, luminometer Victor (Perkin Elmer, Waltham, Massachusetts, USA) was used to determine Wnt/β-catenin promoter activity. The analysis results are shown in FIG. 1.

As can be seen from the test results, escin amplifies Wnt/β-catenin signals by approximately 11 times as compared to the non-treated negative control. The test group including escin shows no concentration-dependent behavior but shows a repetitive clear effect at a concentration of about 20 µg/mL. Particularly, it is shown that escin provides a higher effect as compared to the positive control, wnt3a protein, bound directly to the receptor. Therefore, it can be seen that escin significantly accelerates the activity of the Wnt/β-catenin signal (hair growth-accelerating signal) transmission system, and thus provides an excellent effect of accelerating hair growth.

### Experimental Example 2. Effect of Accelerating Proliferation of Dermal Papilla Cells

Human-derived dermal papilla cells (DPCs) were purchased from PromoCell GmbH. DPCs were cultured under the conditions of 37°C and 5% CO₂ by using a follicle dermal papilla cell growth medium and supplement mix recommended by PromoCell GmbH. The cultured DPCs were pipetted to a 96-well plate at 3,000 cells/well and cultured for 24 hours in the presence of 0.1% of blood serum. Then, the cultured cells were treated with escin at a concentration of 0.2, 0.5, 1.2 and 5 µg/mL for one day and then incubated, and DMSO (vehicle) diluted to 1:1000 in serum-free DMEM was used as a control. After incubation, a cell counting kit (CCK) was used to evaluate proliferation of cells. The culture medium was treated with CCK-8 at a ratio of 1:10, followed by incubation for 1 hour. After 1 hour, the absorbance of each well was determined at 450 nm. All tests were repeated three times and the average of absorbance value was calculated. The results are shown by the percentage of a test group with the result of the control taken as 100. After the test, treatment with escin shows an effect of DPC proliferation, suggesting an excellent effect of proliferation of dermal papilla cells.

**[Table 3]**

| Test Group | Treatment Concentration | Dermal Papilla Cell Proliferation Ability (%) |
|---|---|---|
| Non-treated group | - | 100.0 |
| Escin | 0.2 µg/mL | 168.8 |
| | 0.5 µg/mL | 198.7 |
| | 1 µg/mL | 194.4 |
| | 2 µg/mL | 204.8 |
| | 5 µg/mL | 208.3 |

### Experimental Example 3. Increase in Expression of β-catenin in Dermal papilla Cells

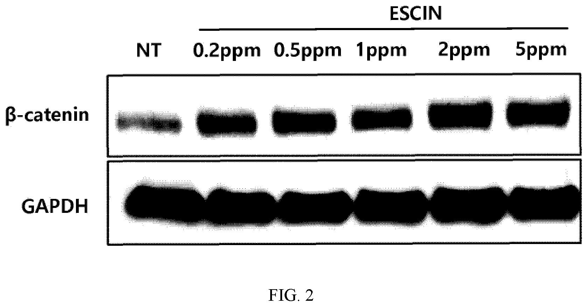

In dermal papilla cells in a catagen stage and a telogen stage, during which Wnt signals are inhibited, β-catenin is decomposed by GSK3-beta. However, in dermal papilla cells in an anagen stage, during which Wnt signals are amplified, activity of GSK3-beta is inhibited and β-catenin is not decomposed, resulting in an increase in expression of β-catenin. Human dermal papilla cells were pipetted to six well plates at about 500,000 cells/well, and treated with escin for 1 day at a concentration of 0.2, 0.5, 1.2 and 5 µg/mL, followed by incubation. After incubation, M-PER lysis buffer was used to cause lysis of the cells and the same amount of cell lysate was loaded on SDS-PAGE gel. The cell lysate loaded on SDS-PAGE gel was transferred to a nitrocellulose membrane and anti-β-catenin antibody was attached to the nitrocellulose membrane to detect protein. GAPDH was used as a control for correcting protein content. The amount of β-catenin increased by escin was determined. The results are shown in FIG. 2.

After the test, it can be seen that the group treated with escin shows a significant increase in amount of β-catenin, which suggests that Wnt/β-catenin signals are increased in dermal papilla cells.

### Experimental Example 4. Determination of Hair Growth Effect of Composition 1 (Hair Tonic) for Treating Alopecia/Accelerating Hair Growth

Composition 1 (hair tonic) for treating alopecia/accelerating hair growth according to the present disclosure was determined in terms of hair growth effect for 105 male and female subjects having a significantly smaller number of hair strands as compared to a normal state or suffering from alopecia and having weak hair. The 105 males and females were divided into 7 groups each including 15 subjects and treated with Comparative Example 1 and Examples 1-6. Each sample was used on hair and scalp five times per week for 6 months. After using each sample, improvement or deterioration was evaluated with a five-grade scoring system in terms of hair thickness, degree of crowding, elasticity and overall evaluation (improvement: +5, +4, +3, +2, +1, no improvement: 0, deterioration: -1, -2, -3, -4, -5). The evaluation was based on the average of the results of examination by interview after using each sample for 6 months. The results are shown in the following Table 4.

**[Table 4]**

| Sample | Hair Thickness | Degree of Crowding | Elasticity | Overall Evaluation |
|---|---|---|---|---|
| Comp. Ex. 1 | -0.5±0.2 | 0.1±0.2 | -0.1±0.2 | -0.21±0.2 |
| Ex. 1 | 2.1±0.2 | 1.5±0.2 | 2.5±0.2 | 2.0±0.2 |
| Ex. 2 | 3.2±0.1 | 2.6±0.2 | 2.6±0.5 | 2.8±0.2 |
| Ex. 3 | 4.5±0.3 | 4.3±0.2 | 3.8±0.3 | 4.2±0.1 |
| Ex. 4 | 4.6±0.2 | 4.3±0.1 | 4.8±0.3 | 4.6±0.2 |
| Ex. 5 | 4.8±0.2 | 4.6±0.1 | 4.3±0.2 | 4.6±0.1 |
| Ex. 6 | 3.1±0.4 | 2.1±0.3 | 2.3±0.6 | 2.5±0.2 |

As can be seen from the above results, Examples 1-6 including escin shows a high effect of preventing alopecia and accelerating hair growth. This demonstrates that the composition for preventing alopecia and accelerating hair growth including escin as an active ingredient according to the present disclosure is significantly effective for treating alopecia.

### Experimental Example 5. Determination of Hair Growth Effect of Composition 1 (Hair Tonic) for Treating Alopecia/Accelerating Hair Growth

Composition 1 (hair tonic) for treating alopecia/accelerating hair growth according to the present disclosure was determined in terms of hair growth effect for 105 male and female subjects having a significantly smaller number of hair strands as compared to a normal state or suffering from alopecia and having weak hair. The 105 males and females were divided into 7 groups each including 15 subjects and treated with Comparative Example 1 and Examples 1-6. Each sample was used on hair and scalp five times per week for 6 months. After using each sample, researcher evaluation through clinical photographs was carried out for Comparative Example and Examples, 2, 4 and 6 months after applying each sample, with a 3-grade evaluation system of 'good', 'slight' and 'no change' (good: 50-70% improved, slight: 20-50% improved, no change). Evaluation of the number of hair strands per unit area and average hair thickness through phototrichogram was carried out for Comparative Example and Examples, 4 and 6 months after applying each composition. The results are shown in the following Table 5.

**[Table 5]**

| Sample | Researcher Evaluation through Clinical Photographs | | | | | | | | | Phototrichogram | | | | | |
|---|---|---|---|---|---|---|---|---|---|---|---|---|---|---|---|
| | No change (person) | | | Slight (person) | | | Good (person) | | | Number of Hair Strands per Unit Area (No./cm²) | | | Average Hair Thickness (µm) | | |
| | 2M | 4M | 6 M | 2M | 4M | 6M | 2M | 4M | 6M | 0M | 4M | 6M | 0M | 4M | 6M |
| Comp. Ex. 1 | 13 | 13 | 12 | 2 | 1 | 2 | 0 | 0 | 1 | 372 ±42 | 375 ±53 | 379 ±24 | 60± 5 | 60± 3 | 62± 2 |
| Ex. 1 | 9 | 8 | 8 | 5 | 6 | 4 | 1 | 1 | 3 | 370 ±27 | 382 ±37 | 397 ±38 | 65± 4 | 65± 5 | 67± 3 |
| Ex. 2 | 8 | 7 | 7 | 4 | 6 | 5 | 3 | 2 | 3 | 327 ±20 | 367 ±39 | 384 ±26 | 60± 4 | 65± 8 | 69± 3 |
| Ex. 3 | 6 | 6 | 4 | 3 | 2 | 3 | 6 | 7 | 8 | 356 ±28 | 364 ±19 | 398 ±22 | 64± 6 | 69± 3 | 76± 7 |
| Ex. 4 | 4 | 4 | 2 | 8 | 6 | 3 | 3 | 5 | 10 | 334 ±33 | 383 ±33 | 403 ±54 | 61± 8 | 64± 3 | 72± 2 |
| Ex. 5 | 3 | 3 | 3 | 8 | 5 | 1 | 4 | 7 | 11 | 322 ±24 | 387 ±46 | 397 ±28 | 64± 7 | 68± 5 | 75± 5 |
| Ex. 6 | 6 | 6 | 5 | 3 | 2 | 3 | 6 | 7 | 7 | 384 ±41 | 394 ±18 | 408 ±47 | 62± 6 | 62± 4 | 69± 5 |

As can be seen from the above results, Examples 1-6 including escin shows a high effect of preventing alopecia and accelerating hair growth. This demonstrates that the composition for preventing alopecia and accelerating hair growth including escin as an active ingredient according to the present disclosure is significantly effective for treating alopecia.

The formulation example, such as hair tonic or hair lotion, is merely an exemplary embodiment of the composition for accelerating hair growth according to the present disclosure, and it is apparent to those skilled in the art that the scope of the composition according to the present disclosure is not limited to the above formulations.

### Preparation Example 3. Composition 3 for Treating Alopecia (Hair Tonic)

At least one of rhaponticin, rhein, chrysophanol and physicon-8-O-glucopyranoside was used to prepare hair tonic in the conventional manner according to the composition of the following Table 6.

**[Table 6]**

| Ingredient s | Weight ratio (%) | | | | | | |
|---|---|---|---|---|---|---|---|
| | Comp. Ex.2 | Comp. Ex.3 | Ex. 8 | Ex. 9 | Ex. 10 | Ex. 11 | Ex. 12 |
| Ethanol | 55 | 55 | 55 | 55 | 55 | 55 | 55 |
| Castor Oil | 5 | 5 | 5 | 5 | 5 | 5 | 5 |
| Glycerin | 3 | 3 | 3 | 3 | 3 | 3 | 3 |
| Active Ingredient | 0 | Minoxidi 12 µg/mL | Rhaponciti n 10 µg/mL | Rhein 10 µg/mL | Chrysophan ol 10 µg/mL | Physicon-8-O-d-glucopyran oside 10 µg/mL | Rhaponcitin, Rhein, Chrysophanol, and Physicon-8-O-d-glucopyranosi de Each 2.5 µg/mL |
| Fragrance and Pigment | q.s. | q.s. | q.s. | q.s. | q.s. | q.s. | q.s. |
| Purified Water | Balance (total 100) | | | | | | |

### Preparation Example 4. Composition 4 for Treating Alopecia (Hair Lotion)

At least one of rhaponticin, rhein, chrysophanol and physicon-8-O-glucopyranoside was used to prepare hair lotion in the conventional manner according to the composition of the following Table 7.

**[Table 7]**

| Ingredients | Weight ratio (%) |
|---|---|
| Cetostearyl alcohol | 2 |
| Stearytriethylammonium chloride | 2 |
| Hydroxyethyl cellulose | 0.5 |
| Rhaponticin, Rhein, Chrysophanol or Physicon-8-O-glucopyranoside | 0.001 |
| Fragrance and Pigment | q.s. |
| Purified Water | Balance (total 100) |

### Experimental Example 6. Effect of Accelerating Proliferation of Dermal Papilla Cells

Human-derived dermal papilla cells (DPCs) were purchased from PromoCell GmbH. The DPCs were cultured in a DMEM medium (Hyclone Inc., UT, USA) containing 5% of fetal bovine serum (FBS; Gibco, NY, USA), 10 units/mL of penicillin and 100 µg/mL of streptomycin at 37°C under 5% of CO₂. The cultured DPCs were pipetted to a 96-well plate at 3,000 cells/well and cultured under the condition of 0.1% of serum for 24 hours. Then, the cultured cells were treated with 10 µg/mL of each of the samples of rhaponticin, rhein, chrysophanol, physicon-8-O-glucopyranoside and combinations thereof as shown in the following Table 8 for one day and then incubated, and DMSO (vehicle) diluted to 1:1000 in serum-free DMEM was used as a control and 2 µg/mL of minoxidil was used as a positive control . After incubation, a cell counting kit (CCK) was used to evaluate proliferation of cells. The culture medium was treated with CCK-8 at a ratio of 1:10, followed by incubation for 1 hour. After 1 hour, the absorbance of each well was determined at 450 nm. All tests were repeated three times and the average of absorbance value was calculated. The results are shown by the percentage of a test group as the result of the control is taken as 100.

After the test, treatment with rhaponticin, rhein, chrysophanol, physicon-8-O-glucopyranoside or a combination thereof shows an excellent effect of accelerating proliferation of dermal papilla cells (Table 8).

**[Table 8]**

| Test Group | Treatment Concentration | Dermal Papilla Cell Proliferation Ability (%) |
|---|---|---|
| Non-treated group | - | 100.0 |
| Minoxidil | 2 µg/mL | 187.2 |
| Rhaponticin | 10 µg/mL | 162.7 |
| Rhein | 10 µg/mL | 152.7 |
| Chrysophanol | 10 µg/mL | 152.0 |
| Physicon-8-O-d-glucopyranoside | 10 µg/mL | 148.3 |
| Rhaponticin and Chrysophanol | each 5 µg/mL | 172.2 |
| Rhein and Chrysophanol | each 5 µg/mL | 168.5 |
| Chrysophanol and Physicon-8-O-d-glucopyranoside | each 5 µg/mL | 160.5 |
| Rhaponticin, Rhein, Chrysophanol and Physicon-8-O-d-glucopyranoside | each 2.5 µg/mL | 189.4 |

### Experimental Example 7. Effect of Controlling Dermal Papilla Cell Activity Signals

In general, it is known that VEGF, IGF-1, FGF, or the like, secreted in dermal papilla cells induce an anagen stage of hair. In addition, versican is known as a marker of an anagen stage of hair and DKK-1 is known as a marker of a catagen stage of hair. Therefore, effects of rhaponticin, rhein, chrysophanol and physicon-O-d-glucopyranoside upon the signals of accelerating an anagen stage and those of inducing a catagen stage in dermal papilla cells were examined according to the present disclosure.

In a 6-well culture plate, dermal papilla cells were seeded to each well at 1x10⁵ and treated with 10 µg/mL of each of the samples of rhaponticin, rhein, chrysophanol, physicon-O-d-glucopyranoside and combinations thereof as shown in the following Table 10. Then, real-time PCR was used to determine expression of each of VEGF, versican and DKK-1 by using Taqman® probe (Thermo Fisher, Massachusetts, USA) as shown in the following Table 9. The results are shown in the following Table 10.

**[Table 9]**

| Genes | Taqman® Probe Analysis ID |
|---|---|
| VEGF | Hs00900055_m1 |
| Versican | Hs00171642_ m1 |
| DKK-1 | Hs00183740m1 |

As a result, it can be seen that rhaponticin, rhein, chrysophanol and physicon-O-d-glucopyranoside increase expression of VEGF, which is a growth factor inducing an anagen stage of hair, and versican as a marker of an anagen stage, and reduce expression of DKK-1, which is a factor inducing a catagen stage of hair (Table 10).

**[Table 10]**

| Test Group/Treatment Concentration | Expression of Genes (%) | | |
|---|---|---|---|
| | VEGF | Versican | DKK-1 |
| Non-treated group | 100 | 100 | 100 |
| Minoxidil 2 µg/mL | 110 | 134 | 128 |
| Rhaponticin 10 µg/mL | 210 | 130 | 112 |
| Rhein 10 µg/mL | 144 | 154 | 76 |
| Chrysophanol 10 µg/mL | 250 | 158 | 123 |
| Physicon-8-O-d-glucopyranoside 10 µg/mL | 164 | 175 | 75 |
| Rhaponticin 5 µg/mL and Chrysophanol 5 µg/mL | 235 | 150 | 111 |
| Rhein 5 µg/mL and Chrysophanol 5 µg/mL | 250 | 158 | 65 |
| Chrysophanol 5 µg/mL and Physicon-8-O-d-glucopyranoside 5 µg/mL | 264 | 192 | 109 |
| Rhaponticin 2.5 µg/mL, Rhein 2.5 µg/mL, Chrysophanol 2.5 µg/mL and Physicon-8-O-d-glucopyranoside 2.5 µg/mL | 295 | 188 | 61 |

It is shown that rhaponticin, rhein, chrysophanol and physicon-O-d-glucopyranoside have excellent effects of inducing hair growth and inhibiting alopecia. The composition for preventing alopecia and accelerating hair growth according to the present disclosure was prepared with various formulations. Particular formulation examples are described herein. However, the formulation examples described herein are for illustrative purposes only, and it is apparent to those skilled in the art that the scope of the present disclosure is not limited to those formulation examples.

### Experimental Example 8. Test for Determining Effect of Hair Growth of Composition 3 for Treating Alopecia (Hair Tonic)

Composition 3 (hair tonic) for treating alopecia obtained from Preparation Example 3 was used for 40 male and female subjects having a significantly smaller number of hair strands as compared to a normal state or suffering from alopecia and having weak hair. The 40 males and females were divided into 4 groups each including 10 subjects and treated with Comparative Examples 2 and 3 and Examples 8-12. Each sample was used on hair and scalp five times per week for 6 months. After using each sample, improvement was evaluated in terms of hair thickness, degree of crowding, elasticity and overall evaluation (significantly improved: +3, improved: +2, slightly improved: +1, no change: 0, slightly deteriorated: -1, deteriorated: -2, significantly deteriorated: -3). The evaluation was based on the average of the results of examination by interview after using each sample for 6 months. The results are shown in the following Table 11.

**[Table 11]**

| Sample | Thickness of Hair | Degree of Crowding | Elasticity | Overall Evaluation |
|---|---|---|---|---|
| Comp. Ex. 2 | -0.5±0.2 | 0.1±0.2 | -0.1±0.2 | -0.2.1±0.2 |
| Comp. Ex. 3 | 2.1±0.2 | 2.3±0.1 | 2.1±0.3 | 2.1±0.2 |
| Ex. 8 | 2.8±0.3 | 2.7±0.1 | 2.5±0.4 | 2.8±0.1 |
| Ex. 9 | 2.6±0.1 | 2.8±0.2 | 2.4±0.3 | 2.7±0.2 |
| Ex. 10 | 1.9±0.1 | 2.6±0.3 | 2.0±0.2 | 2.3±0.1 |
| Ex. 11 | 2.6±0.3 | 2.1±0.1 | 2.4±0.1 | 2.6±0.1 |
| Ex. 12 | 2.7±0.3 | 2.9±0.3 | 2.7±0.3 | 2.8±0.3 |

As can be seen from the above results, Examples using rhaponticin, rhein, chrysophanol and physicon-O-d-glucopyranoside show high effects of preventing alopecia and accelerating hair growth. Thus, it can be seen that the composition according to the present disclosure is significantly effective for treating alopecia.

## Claims

1. A composition for preventing alopecia or accelerating hair growth, comprising escin as an active ingredient.

2. The composition for preventing alopecia or accelerating hair growth according to claim 1, wherein the content of the active ingredient is 0.001-10 wt%, based on the total weight of the composition.

3. A composition for preventing alopecia or accelerating hair growth, comprising at least one compound selected from rhaponticin, rhein, chrysophanol and physicon-8-O-d-glucopyranoside, or a salt, hydrate or solvate thereof, as an active ingredient.

4. The composition for preventing alopecia or accelerating hair growth according to claim 3, which comprises at least two of the compounds.

5. The composition for preventing alopecia or accelerating hair growth according to claim 3, wherein the compound comprises: (i) chrysophanol; and (ii) at least one compound selected from rhein, rhaponticin and physicon-8-O-d-glucopyranoside.

6. The composition for preventing alopecia or accelerating hair growth according to claim 3, wherein the compound comprises rhaponticin, rhein, chrysophanol and physicon-8-O-d-glucopyranoside.

7. The composition for preventing alopecia or accelerating hair growth according to claim 3, wherein the content of the active ingredient is 0.00001-50 wt%, based on the total weight of the composition.

8. The composition for preventing alopecia or accelerating hair growth according to claim 1 or 3, which accelerates proliferation of dermal papilla cells.

9. The composition for preventing alopecia or accelerating hair growth according to claim 1 or 3, which enhances hair elasticity.

10. The composition for preventing alopecia or accelerating hair growth according to claim 1 or 3, which is a pharmaceutical composition.

11. The composition for preventing alopecia or accelerating hair growth according to claim 1 or 3, which is a non-medical composition.

12. The composition for preventing alopecia or accelerating hair growth according to claim 1 or 3, which is a cosmetic composition.

13. A hair or scalp care product comprising the composition for preventing alopecia or accelerating hair growth as defined in claim 1 or 3.

14. The hair or scalp care product according to claim 13, which is selected from the group consisting of a hair growth treatment, scalp clinic agent, scalp scaling agent, scalp massaging agent, scalp care agent, cleaner, shampoo, tonic, hair conditioner, hair lotion, gel, pack, cream, essence, powder, spray, oil, soap, ointment, hair styling agent, hair dye and hair perm agent.
